Europäisches Patentamt

(19) European Patent Office

Office européen des brevets

(11) Numéro de publication: **0 227 537 B1**

(12) **FASCICULE DE BREVET EUROPEEN**

(45) Date de publication du fascicule du brevet:
21.03.90

(21) Numéro de dépôt: **86402746.1**

(22) Date de dépôt: **10.12.86**

(51) Int. Cl.⁴: **A61M 1/12**

(54) **Prothèse cardiaque totale comportant deux pompes découplées associées en une unité fonctionnellement indissociable.**

(30) Priorité: **12.12.85 FR 8518425**

(43) Date de publication de la demande:
**01.07.87 Bulletin 87/27**

(45) Mention de la délivrance du brevet:
**21.03.90 Bulletin 90/12**

(84) Etats contractants désignés:
**DE GB IT**

(56) Documents cités:
**EP-A- 0 146 445**
**WO-A-85/02339**
**US-A- 4 173 796**

(73) Titulaire: **AEROSPATIALE Société Nationale Industrielle, 37, Boulevard de Montmorency, F-75781 Paris Cédex 16(FR)**

(72) Inventeur: **Chareire, Jean-Louis, 66, rue Aristide Briand, F-92300 Levallois(FR)**
Inventeur: **Lapeyre, Didier, Chaignes, F-27120 Pacy-sur-Eure(FR)**

(74) Mandataire: **Bonnetat, Christian et al, CABINET BONNETAT 23, Rue de Léningrad, F-75008 Paris(FR)**

ACTORUM AG

## Description

Dans la demande de brevet EP-A 0 146 445 on a décrit une prothèse cardiaque totale comprenant deux pompes, respectivement représentatives du coeur droit et du coeur gauche, ainsi qu'un dispositif de commande desdites pompes, cette prothèse étant remarquable en ce que, d'une part, elle comporte l'unité fonctionnelle indissociable constituée :

- d'un module péricardique, destiné à être logé dans la cavité du coeur naturel à remplacer et enfermé dans une enveloppe étanche portant quatre orifices de raccord destinés respectivement à être raccordés à l'oreillette droite, à l'artère pulmonaire, à l'oreillette gauche et à l'aorte, lesdits orifices de raccord à l'oreillette droite et à l'artère pulmonaire étant pourvus de valves pour servir respectivement d'orifice d'entrée et d'orifice de sortie à une première pompe logée dans ledit module péricardique et destinée à accomplir la fonction du coeur droit du coeur naturel à remplacer;

- d'un module extra-péricardique, destiné à être logé dans un espace physiologiquement neutre du malade receveur et à accomplir la fonction du coeur gauche du coeur naturel à remplacer, ce module extra-péricardique comportant une enveloppe étanche dans laquelle est enfermée une seconde pompe pourvue d'un orifice d'entrée et d'un orifice de sortie, pourvus chacun d'une valve;

- d'une liaison fonctionnelle entre lesdits modules péricardique et extra-péricardique comportant :

- un premier conduit traversant l'enveloppe dudit module péricardique et réunissant l'orifice de celui-ci correspondant à l'oreillette gauche et l'orifice d'entrée de ladite seconde pompe incorporée dans ledit module extra-péricardique;

- un second conduit traversant l'enveloppe dudit module péricardique et réunissant l'orifice de celui-ci correspondant à l'aorte et l'orifice de sortie de ladite seconde pompe incorporée dans ledit module extra-péricardique;

- un troisième conduit établissant une communication gazeuse entre les côtés desdites première et seconde pompes opposés au sang traversant celles-ci;

et en ce que, d'autre part, ledit dispositif de commande actionne lesdites pompes en opposition.

Ainsi, grâce à la structure éclatée, quoique monobloc et indissociable, de la prothèse selon l'invention, seul ledit module péricardique, c'est-à-dire le coeur droit, se trouve dans la cavité péricardique. On dispose alors de l'emplacement nécessaire pour y loger une enveloppe enfermant une pompe et son système électro-mécanique d'actionnement, faisant office de ventricule droit.

Cette enveloppe dudit module péricardique présente, au moins approximativement, la forme du coeur naturel malade à remplacer, et sur ladite enveloppe, la disposition des orifices de raccord à l'oreillette droite, à l'artère pulmonaire, à l'oreillette gauche et à l'aorte correspond au moins sensiblement à la disposition naturelle de ces oreillettes et artères. En effet, la place dégagée dans la cavité péricardique par l'éloignement de la pompe faisant office de coeur gauche, permet d'agencer ledit module péricardique de façon que la jonction aux veines et artères soit aussi optimale que possible.

Dans une telle prothèse cardiaque, c'est la partie thoracique de l'aorte qui est reliée au module péricardique, de sorte que l'on prévoit une liaison artificielle (le second conduit) entre les deux modules pour prolonger l'aorte.

Dans certains cas, plutôt que de prévoir une telle liaison artificielle, il peut être préférable d'utiliser l'aorte elle-même.

L'objet de la présente invention est de permettre une telle utilisation.

A cette fin, selon l'invention, la prothèse cardiaque totale comprend d'une part, l'unité fonctionnelle indissociable constituée :

- d'un module péricardique, destiné à être logé dans la cavité du coeur naturel à remplacer et enfermé dans une enveloppe étanche portant seulement trois orifices de raccord destinés respectivement à être raccordés à l'oreillette droite, à l'artère pulmonaire et à l'oreillette gauche, lesdits orifices de raccord à l'oreillette droite et à l'artère pulmonaire étant pourvus de valves pour servir respectivement d'orifice d'entrée et d'orifice de sortie à une première pompe logée dans ladite enveloppe et destinée à accomplir la fonction du coeur droit du coeur naturel à remplacer;

- d'un module extra-péricardique, destiné à être logé dans un espace de la cavité abdominale du malade receveur et à accomplir la fonction de coeur gauche du coeur naturel à remplacer, ce module extra-péricardique comportant une seconde pompe enfermée dans une enveloppe étanche, pourvue d'un orifice d'entrée et d'un orifice de sortie, équipés chacun d'une valve, ledit orifice de sortie étant destiné à être raccordé à la partie abdominale de l'aorte;

- d'une liaison fonctionnelle entre lesdits modules comportant seulement:

- un premier tube traversant ladite enveloppe dudit module péricardique et réunissant l'orifice de celui-ci correspondant à l'oreillette gauche et l'orifice d'entrée de la seconde pompe incorporée dans ledit module extra-péricardique;

- un second tube établissant une communication gazeuse entre les côtés desdites première et seconde pompes opposés au sang traversant celle-ci;

et d'autre part, un dispositif de commande qui actionne lesdites pompes en opposition.

Ainsi, selon l'invention, par rapport à la prothèse mentionnée ci-dessus, on peut supprimer l'orifice du module péricardique destiné à être relié à l'aorte, ainsi que ledit second conduit, les premier et second tubes de la prothèse selon la présente invention correspondant respectivement au premier conduit et au troisième conduit de la prothèse antérieure.

La prothèse conforme à la présente invention peut donc présenter tous les avantages de cette prothèse antérieure, à savoir :

A- Elle ne perturbe en rien le retour sanguin veineux par les veines caves et les veines pulmonaires et il est alors possible de régler la fréquence de battement de la prothèse en fonction de la pression de retour du sang dans le coeur droit, ce qui est particulièrement avantageux. Par suite, un capteur peut être disposé dans ledit module péricardique pour détecter la pression du sang veineux entrant dans ladite première pompe et réguler le fonctionnement de la prothèse en fonction de la pression mesurée.

B - L'éloignement du coeur gauche de la cavité péricardique permet de ménager suffisamment d'espace pour loger, dans ledit module péricardique, un système électromécanique d'actionnement de ladite première pompe, ainsi que les mécanismes de commande de valves électrocommandées.

C - Ladite première pompe peut être du type à membrane et ledit système électro-mécanique peut alors être du type à plateau-poussoir en contact direct avec ladite membrane. Il est donc possible d'optimaliser les qualités biologiques de la prothèse selon l'invention, en conformant ledit plateau pour qu'il entraîne le minimum de contraintes mécaniques à l'origine de la formation de microfissures dans la membrane, ces microfissures étant de façon connue, à l'origine de la dégradation de la qualité de la membrane motrice de la prothèse. De plus, le capteur, mentionné ci-dessus et destiné à réguler le fonctionnement de la prothèse, peut alors être monté sur la membrane de ladite première pompe.

D- Puisque le module extra-péricardique est logé dans un volume abdominal aisément accessible au prix d'un opération chirurgicale mineure, ledit module extra-péricardique, qui se trouve être le module de puissance et donc celui qui est le plus sujet à usure, peut en cas de panne, être aisément remplacé par un nouveau module à fonctionnement irréprochable.

Pour faciliter un tel échange, il est avantageux que l'enveloppe dudit module extra-péricardique porte des orifices de raccord respectivement reliés à l'orifice d'entrée, à l'orifice de sortie et au côté de ladite seconde pompe opposé au sang et pouvant être facilement et respectivement branchés et débranchés dudit premier tube, du raccord à l'aorte abdominale et dudit second tube.

E - Puisque le volume de l'espace abdominal peut être relativement important (jusqu'à un litre), il ne se pose aucun problème d'encombrement, et ledit module extra-péricardique peut incorporer également un système électromécanique à rendement élevé pour l'actionnement direct de ladite seconde pompe. Celle-ci peut également être du type à membrane, de sorte que le système électro-mécanique associé est alors du type à plateau-poussoir en contact direct avec cette membrane.

F - Pour la raison qui précède, chacune des deux valves incorporées dans ledit module extra-péricardique peut être du type à ouverture et fermeture commandées, comme celles prévues pour ledit module péricardique. Il est alors avantageux que le dispositif de commande de la prothèse comporte un microprocesseur commandant les systèmes électromécaniques incorporés dans lesdits modules, ainsi que les quatre valves de ceux-ci. Ce microprocesseur reçoit alors les informations du capteur disposé dans ledit module péricardique et permettant de régler le rythme cardiaque aux besoins du malade.

De préférence, ladite liaison réunit la partie inférieure de l'enveloppe dudit module péricardique à la partie supérieure de l'enveloppe dudit module extra-péricardique.

Selon une caractéristique importante de la présente invention, ledit second tube de ladite liaison entre lesdits modules péricardique et extra-péricardique est souple longitudinalement, mais rigide radialement et il enferme ledit premier tube.

Ainsi, ce premier tube est protégé mécaniquement par le second. Ceci est particulièrement avantageux, du fait que ledit premier tube doit être très souple, donc très mince, et ne subir aucune perte de charge provoquée. Ledit premier tube, associé à l'espace intérieur dudit second tube, fait alors fonction d'oreillette gauche complémentaire. On remarquera que, grâce audit second tube, ladite liaison entre les modules sert de chambre de compliance pour l'ensemble de la prothèse.

Dans ce second tube, on peut faire passer un câble électrique reliant lesdits modules péricardique et extra-péricardique. Ainsi, la commande du système électromécanique et des valves dudit module péricardique peut passer par ledit module extra-péricardique et ledit câble électrique contenu dans la liaison.

Les figures du dessin annexé feront bien comprendre comment l'invention peut être réalisée. Sur ces figures, des références identiques désignent des éléments semblables. De plus, sur ces figures, des éléments identiques à ceux représentés sur les figures de la demande de brevet EP-A 0 146 445 portent des références identiques.

La figure 1 montre schématiquement un coeur naturel en liaison avec ses veines et artères principales, en vue antérieure.

La figure 2 est un schéma de fonctionnement du coeur de la figure 1.

La figure 3 illustre schématiquement la prothèse cardiaque décrite dans la demande de brevet mentionnée ci-dessus.

La figure 4 donne le schéma de la prothèse cardiaque selon la présente invention.

La figure 5 illustre schématiquement la prothèse selon l'invention en place sur un patient.

La figure 6 montre schématiquement, de l'extérieur, un mode de réalisation du module péricardique et le départ de ladite liaison entre les modules.

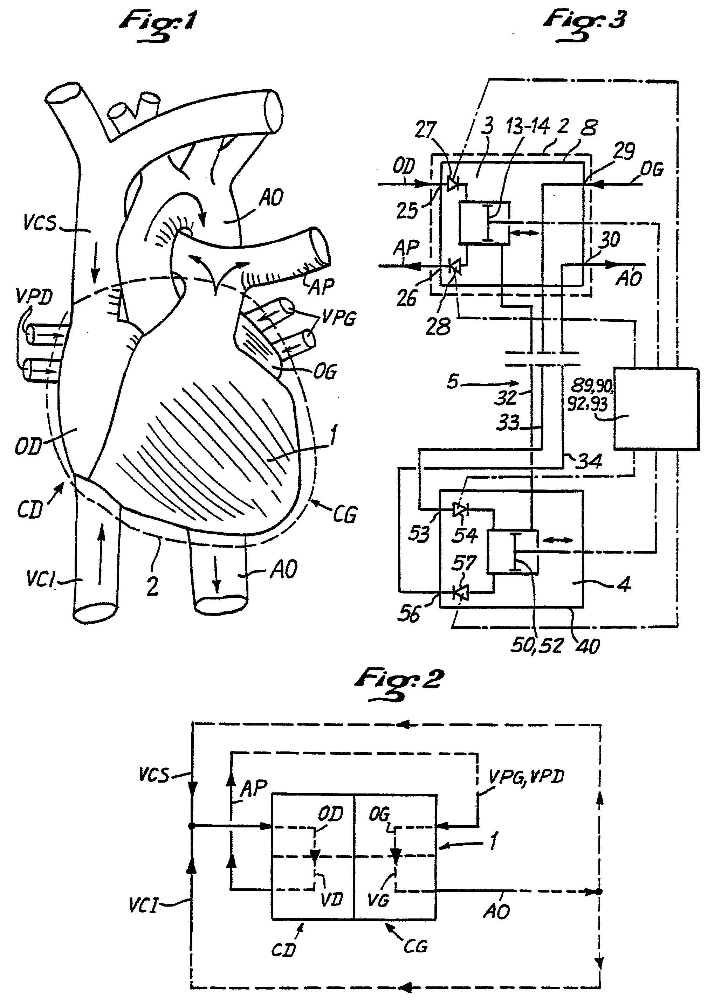

Comme illustré schématiquement sur les figures 1 et 2, un coeur humain naturel 1 est logé dans la cavité péricardique 2 (simplement illustrée par un trait tireté 2) et est composé en réalité de deux coeurs distincts, mais solidaires l'un de l'autre, à savoir le coeur droit CD comportant l'oreillette droite OD et le ventricule droit VD et le coeur gauche CG comportant l'oreillette gauche OG et le ventricule gauche VG. L'oreillette OD du coeur droit CD reçoit le sang veineux par la veine cave supérieure VCS et par la veine cave inférieure VCI, tandis que le ventricule VD dudit coeur droit CD fait passer le sang ainsi reçu vers les poumons par l'intermédiaire de l'artère pulmonaire AP.

De même, l'oreillette OG du coeur gauche CG reçoit le sang provenant des poumons par les veines pulmonaires gauches VPG et droites VPD et le ventricule VG du coeur gauche CG chasse le sang reçu par l'aorte AO.

L'idée de base de la prothèse à pompes découplées du type auquel se rapporte la présente invention repose sur la constatation physiologique que, quoique constitué de deux pompes CD et CG formant une unité musculaire unique, le coeur 1 est en réalité composé de deux ensembles fonctionnellement indépendants. En effet, sur le plan fonctionnel, le coeur droit CD peut être considéré comme un simple coeur de passage qui pousse une colonne sanguine dont la vitesse d'écoulement est variable, mais jamais nulle, sauf lorsque les fréquences des battements du coeur 1 sont très basses. Lorsque le débit sanguin du système vasculaire augmente par suite d'une augmentation de fréquence de ces battements, la participation du coeur droit CD à la mise en mouvement du sang dans le circuit pulmonaire diminue du fait de l'augmentation de la vitesse, et donc de l'énergie cinétique, du sang parvenant au coeur droit CD. En revanche, le coeur gauche CG, par son puissant ventricule, constitue le coeur proprement dit, c'est-à-dire la pompe propulsive chargée d'assurer la perfusion sanguine de tous les organes et tissus de l'organisme.

De plus, cette idée de base s'appuie sur le fait connu de l'homme de l'art que la contraction du ventricule du coeur droit CD et celle du ventricule du coeur gauche CG peuvent être non pas simultanées, mais en opposition de phase.

Comme l'illustre très schématiquement la figure 3, la prothèse cardiaque totale décrite dans la demande de brevet rappelée ci-dessus est constituée d'une unité fonctionnellement indissociable constituée de deux modules de pompage 3 et 4 découplés, mais reliés l'un à l'autre par une liaison fonctionnelle tubulaire 5.

Le module de pompage 3, destiné à remplacer le coeur droit CD du coeur naturel 1, est logé dans la cavité péricardique 2. Il comporte une enveloppe étanche 8 sur laquelle sont montés quatre embouts de raccord de tout type connu 25,26,29 et 30, respectivement destinés à le raccorder à l'oreillette droite OD (réservoir des veines caves VCI et VCS), à l'artère pulmonaire AP, à l'oreillette gauche OG (réservoir des veines pulmonaires VPG et VPD) et à l'aorte AO, après coupe de celles-ci et exérèse des ventricules naturels de la cavité péricardique 2.

Les orifices 25 et 26 de raccord à l'oreillette droite OD et à l'artère pulmonaire AP sont pourvus de valves 27,28 pour servir respectivement d'orifice d'entrée et d'orifice de sortie à une pompe 13,14 logée dans l'enveloppe 8 et destinée à accomplir la fonction du coeur droit du coeur naturel à remplacer.

Le module de pompage 4, destiné à jouer le rôle du coeur gauche CG du coeur naturel 1, est logé à l'extérieur de la cavité péricardique 2, dans un espace physiologiquement neutre, par exemple du thorax ou de l'abdomen. Il comporte une enveloppe étanche 40 enfermant une pompe 50,52 pourvue d'un orifice d'entrée 53 et d'un orifice de sortie 56, équipés chacun d'une valve 54 ou 57.

La liaison fonctionnelle 5, qui peut sans inconvénient traverser le diaphragme du patient recevant la prothèse , comporte :

- un premier conduit 33 assurant la connexion entre l'orifice 29 du module péricardique 3 correspondant à l'oreillette gauche OG et l'orifice d'entrée 53 de la pompe 50,52 incorporée dans le module extra-péricardique 4;
- un second conduit 34 assurant la connexion entre l'orifice 30 du module péricardique 3 correspondant à l'aorte AO et l'orifice de sortie 56 de ladite pompe 50,52 incorporée dans ledit module extra-péricardique 4;
- un troisième conduit 32 établissant une communication gazeuse entre les côtés des pompes 13,14 et 50,52 opposés au sang traversant celles-ci.

En outre, un dispositif de commande 89,90,92,93 actionne les pompes 13,14 et 50,52 en opposion et contrôle les valves 27,28,54 et 57

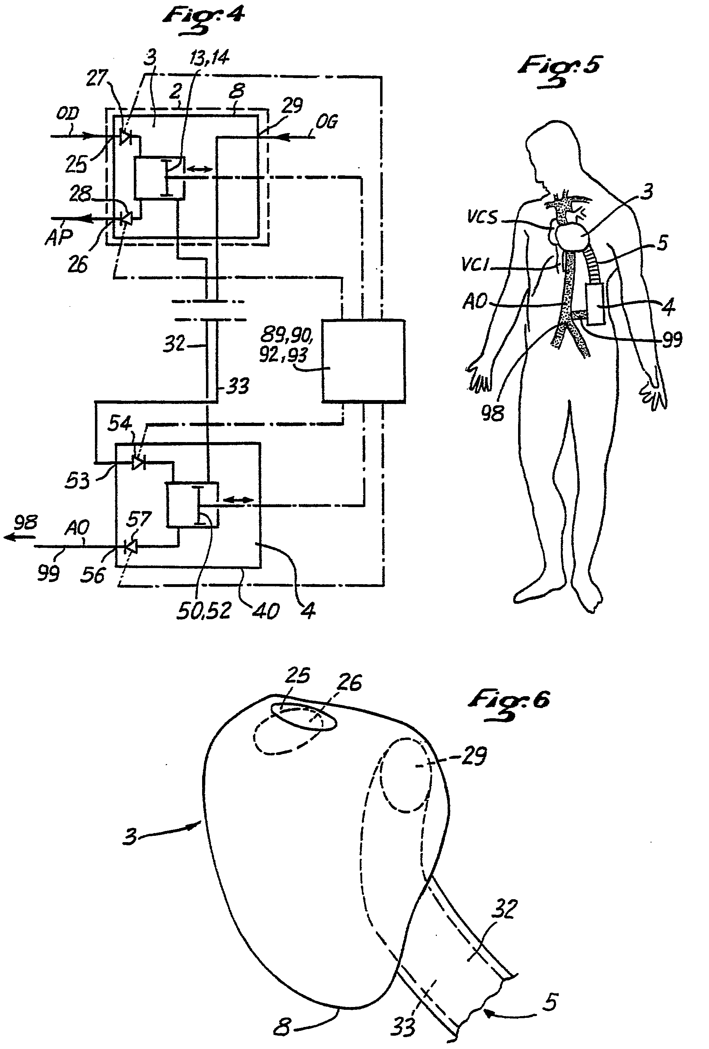

Sur la figure 4 on a représenté, selon un schéma comparable à celui de la figure 3, la prothèse selon la présente invention. Cette prothèse est en tous points semblable à celle de la figure 3, excepté le fait que l'enveloppe 8 du module péricardique 3 ne comporte pas d'orifice 30 et que la liaison 5 ne comporte pas de conduit 34.

Dans ce cas (voir également la figure 5), l'orifice 56 du module extra-péricardique 4 est directement relié à la partie abdominale 98 de l'aorte AO par un raccord 99.

Bien entendu, quoique cela ne soit pas représenté sur les figures, les orifices 25,26,29 et 56 sont pourvus de dispositifs de raccord.

Comme le montre la figure 6, le tube 33 est enfermé dans le tube 32 et, du côté du module péricardique 3, ces deux tubes peuvent être solidaires de celui-ci. En revanche, du côté du module abdominal 4, on peut prévoir un raccord rapide (non représenté).

Le tube 32 présente une souplesse longitudinale suffisante pour lui permettre de s'adapter par flexion,sans coudure ni écrasement, au mieux au passage physiologique pouvant exister entre la cavité péricardique 2 dans laquelle est disposé le module de pompage 3 et la cavité abdominale dans laquelle est placé le module de pompage 4. En revanche, le tube 32 présente une grande rigidité radiale, afin d'éviter toute formation de plis et toute compression extérieure. Ce tube 32 peut par exemple comporter à cet effet, dans sa paroi, un fil spiralé ou analogue. Le diamètre de l'enveloppe tubulaire 32 peut être de l'ordre de 5cm.

A l'intérieur dudit tube 32, comme il a été mentionné ci-dessus, passe le tube souple 33 prolongeant en une veine pulmonaire commune l'oreillette gauche dans laquelle débouchent les veines pulmonaires gauches et droites et une connexion électrique (non représentée) reliant le module de pompage 3 au module de pompage 4 et à un générateur électrique (non représenté). La connexion électrique permet l'alimentation et l'asservissement du moteur d'actionnement de la pompe 13,14 et des valves électrocommandées disposées dans les orifices 25 et 26.

Le tube souple 33 doit présenter une section supérieure à 8 cm2, pour éviter les pertes de charge.

Le tube 33 et ladite connexion électrique laissent subsister, à l'intérieur de l'enveloppe tubulaire 32, un espace libre mettant en communication gazeuse les espaces morts des deux modules de pompage 3 et 4.

A propos de la structure de la liaison 5, on peut faire les remarques suivantes :

1 - Pour être adapté à l'oreillette gauche et aux veines pulmonaires, le tube 33 doit être très souple et présenter une consistance identique, voisine de la baudruche. Le tube 33 est donc très fragile. Cependant, il n'en résulte aucun inconvénient puisque, dans la liaison 5, le tuyau 33 est bien protégé par le tube 32 plus rigide.

2- L'espace intérieur libre dans le tube 32 met en communication les espaces morts des modules de pompage 3 et 4. Ceux-ci fonctionnant en opposition, ledit espace intérieur libre permet donc au volume gazeux chassé par la pompe d'un desdits modules, de se déplacer vers l'autre de ces derniers.

Il sert donc de chambre de compensation de volume nécessitée par le fonctionnement de pompes à membrane à plateau-poussoir. De plus, il permet d'équilibrer les variations de pression atmosphérique puisque le sang contenu dans la veine pulmonaire commune 33 est en équilibre avec la pression atmosphérique. Cet espace intérieur résoud donc de façon élégante le délicat problème de la chambre de "compliance",mentionné par de nombreux auteurs.

3 - La pompe du module de puissance 4 extra-péricardique risque de provoquer des coups de boutoir hydrauliques dans le tuyau 33 prolongeant l'oreillette gauche. Cependant, il n'en résulte aucun inconvénient. En effet, en cas de coups de boutoir hydrauliques, le tube 33, dont la consistance est très souple, se dilate radialement, ce qu'il peut faire grâce à l'existence de l'espace libre pneumatique à l'intérieur du tube 32. Ainsi, le tube 33 en association avec cet espace libre joue le rôle d'un réservoir de sang, à la manière d'une oreillette: il peut donc être considéré comme une oreillette gauche complémentaire.

4 - Eventuellement, pour compléter la fonction tampon dudit espace libre, on peut faire en sorte que, à l'intérieur du module péricardique 3, l'enveloppe ou la paroi (ce peut être une partie de l'enveloppe 8) entourant le tube 33 soit souple, au lieu d'être rigide.

Sur la figure 6, on peut voir que l'enveloppe 8 du module péricardique 3 peut présenter au moins approximativement la forme, le volume et la masse du coeur naturel 1 à remplacer et que, sur ladite enveloppe, la disposition des orifices 25,26 et 29 de raccord à l'oreillette droite, à l'artère pulmonaire et à l'oreillette gauche correspond au moins sensiblement à la disposition naturelle de ces oreillettes et artère .

## Revendications

1. Prothèse cardiaque totale comprenant d'une part, l'unité fonctionnelle indissociable constituée :
- d'un module péricardique (3) destiné à être logé dans la cavité (2) du coeur naturel (1) à remplacer et enfermé dans une enveloppe étanche (8) portant seulement trois orifices de raccord (25), (26) et (29) destinés respectivement à être raccordés à l'oreillette droite OD, à l'artère pulmonaire AP et à l'oreillette gauche OG, lesdits orifices (25) et (26) de raccord à l'oreillette droite OD et à l'artère pulmonaire AP étant pourvus de valves pour servir respectivement d'orifice d'entrée et d'orifice de sortie à une première pompe (13,14) logée dans ladite enveloppe (8) et destinée à accomplir la fonction du coeur droit du coeur naturel à remplacer;
– d'un module extra-péricardique (4), destiné à être logé dans un espace de la cavité abdominale du malade receveur et à accomplir la fonction du coeur gauche du coeur naturel (1) à remplacer, ce module extra-péricardique comportant une seconde pompe (50,52) enfermée dans une enveloppe étanche (40) pourvue d'un orifice d'entrée (53) et d'un orifice de sortie (56), équipés chacun d'une valve, ledit orifice de sortie (56) étant destiné à être raccordé à la partie abdominale de l'aorte;
– d'une liaison fonctionnelle (5) entre lesdits modules comportant seulement:
   – un premier tube (33) traversant ladite enveloppe (8) dudit module péricardique (3) et réunissant l'orifice (29) de celui-ci correspondant à l'oreillette gauche OG et l'orifice d'entrée (53) de la seconde pompe (50,52) incorporée dans ledit module extra-péricardique (4);
   – un second tube (32) établissant une communication gazeuse entre les côtés desdites première et seconde pompes opposés au sang traversant celles-ci;
   et, d'autre part, un dispositif de commande (89,90,92,93) qui actionne lesdites pompes en opposition.

2. Prothèse cardiaque totale selon la revendication 1, caractérisée en ce que ledit module péricardique (3) enferme un système électromécanique d'actionnement de ladite première pompe (13,14).

3. Prothèse cardiaque totale selon l'une des revendications 1 ou 2,
caractérisée en ce que ladite enveloppe (8) dudit module péricardique (3) présente, au moins approximativement, la forme, le volume et la masse du coeur naturel à remplacer et en ce que, sur ladite enveloppe (8), la disposition des orifices (25,26 et 29) de raccord à l'oreillette droite OD, à l'artère pulmonaire AP, et à l'oreillette gauche OG correspond au moins sensiblement à la disposition naturelle de ces oreillettes et artère.

4. Prothèse cardiaque totale selon l'une des revendications 1 à 3,
caractérisée en ce que, dans ledit module péricardique (3), les valves prévues dans les orifices (25,26) de raccord à l'oreillette droite et à l'artère pulmonaire sont du type électro-commandé.

5. Prothèse cardiaque totale selon l'une des revendications 1 à 4,
caractérisée en ce que ladite première pompe (13,14) est du type à membrane et plateau-poussoir.

6. Prothèse cardiaque totale selon l'une quelconque des revendications 1 à 5,
caractérisée en ce que ladite liaison (5) réunit la partie inférieure de ladite enveloppe (8) dudit module péricardique (3) à la partie supérieure de ladite enveloppe (40) dudit module extra-péricardique (4).

7. Prothèse cardiaque totale selon l'une quelconque des revendications 1 à 6,
caractérisée en ce que ledit second tube (32) de ladite liaison (5) entre les modules est souple longitudinalement, mais rigide radialement et enferme ledit premier tube (33).

8. Prothèse cardiaque totale selon l'une quelconque des revendications 1 à 7,
caractérisée en ce que ladite seconde pompe (50,52) est du type à membrane et à plateau-poussoir.

9. Prothèse cardiaque totale selon l'une quelconque des revendications 1 à 8,
caractérisée en ce que chacune des deux valves (54,57) incorporée audit module extra-péricardique (4) est du type électrocommandé.

## Patentansprüche

1. Totale Herzprothese mit, einerseits, einer funktionellen Einheit, bestehend aus:
   - einem perikardialen Modul (3), der dazu bestimmt ist, in der Höhle (2) des zu ersetzenden natürlichen Herzens (1) untergebracht und in eine dichte Umhüllung (8) eingeschlossen zu werden, und nur drei Anschlußöffnungen (25, 26 und 29) aufweist, die dazu bestimmt sind, an die rechte Vorkammer OD, an die Lungenarterie AP und an die linke Vorkammer OG angeschlossen zu werden, wobei die Öffnungen (25 und 26) zum Anschluß der rechten Vorkammer OD und der Lungenarterie AP mit Ventilen ausgestattet sind, wodurch sie als Einlaßöffnung bzw. Auslaßöffnung einer ersten Pumpe (13, 14) dienen können, welche in der Umhüllung (8) untergebracht und dazu bestimmt ist, die Funktion des rechten Herzens des zu ersetzenden natürlichen Herzens (1) zu erfüllen;
   - einem extra-perikardialen Modul (4), der dazu bestimmt ist, an einer Stelle der Bauchhöhle des kranken Empfängers untergebracht zu werden und die Funktion des linken Herzens des zu ersetzenden natürlichen Herzens (1) zu erfüllen, wobei dieser extra-perikardiale Modul eine in eine dichte Umhüllung (40) eingeschlossene zweite Pumpe (50, 52) enthält, die eine Einlaßöffnung (53) und eine Auslaßöffnung (56) aufweist, von denen jede mit einem Ventil ausgestattet ist, und wobei die Auslaßöffnung (56) dazu bestimmt ist, an die abdominale Partie der Aorta angeschlossen zu werden;
   - einer funktionellen Verbindung (5) zwischen den Modulen (3 und 4) mit nur:
     - einem ersten Kanal (33), der die Umhüllung (8) des perikardialen Moduls (3) durchsetzt und die der linken Vorkammer OG entsprechende Öffnung (29) desselben mit der Einlaßöffnung (53) der zweiten, in dem extra-perikardialen Modul (4) enthaltenen Pumpe (50, 52) verbindet;
     - einem zweiten Kanal (32), der eine gasleitende Verbindung zwischen den Seiten der ersten und zweiten Pumpe herstellt, die dem diese durchfließenden Blut entgegengesetzt sind; und, andererseits, einer Steuervorrichtung (89, 90, 92, 93), welche diese Pumpen im Gegentakt betätigt.

2. Totale Herzprothese nach Anspruch 1, dadurch gekennzeichnet, daß der perikardiale Modul (3) ein elektromechanisches System zur Betätigung der ersten Pumpe (13, 14) umschließt.

3. Totale Herzprothese nach einem der Ansprüche 1 oder 2, dadurch gekennzeichnet, daß die Umhüllung (8) des perikardialen Moduls (3) wenigstens annähernd die Form, das Volumen und die Masse des zu ersetzenden natürlichen Herzens (1) aufweist, und daß die Anordnung der Öffnungen (25, 26 und 29) zum Anschluß der rechten Vorkammer OD, der Lungenarterie AP und der linken Vorkammer OG wenigstens einigermaßen der natürlichen Anordnung dieser Vorkammern und dieser Arterie entspricht.

4. Totale Herzprothese nach einem der Ansprüche 1 bis 3, dadurch gekennzeichnet, daß in dem perikardialen Modul (3) die in den Öffnungen (25, 26) zum Anschluß der rechten Vorkammer und der Lungenarterie vorgesehenen Ventile elektrisch steuerbar sind.

5. Totale Herzprothese nach einem der Ansprüche 1 bis 4, dadurch gekennzeichnet, daß die erste Pumpe (13, 14) als Membranpumpe mit Stoßplatte ausgebildet ist.

6. Totale Herzprothese nach einem der Ansprüche 1 bis 5, dadurch gekennzeichnet, daß die Verbindung (5) die untere Partie der Umhüllung (8) des perikardialen Moduls (3) mit der oberen Partie der Umhüllung (40) des extra-perikardialen Moduls (4) verbindet.

7. Totale Herzprothese nach einem der Ansprüche 1 bis 6, dadurch gekennzeichnet, daß der zweite Kanal (32) der Verbindung (5) zwischen den Modulen in Längsrichtung biegsam, aber in radialer Richtung steif ist und den ersten Kanal (33) umschließt.

8. Totale Herzprothese nach einem der Ansprüche 1 bis 7, dadurch gekennzeichnet, daß die zweite Pumpe (50, 52) als Membranpumpe mit Stoßplatte ausgebildet ist.

9. Totale Herzprothese nach einem der Ansprüche 1 bis 8, dadurch gekennzeichnet, daß jedes der zwei Ventile (54, 57), die in dem extra-perikardialen Modul (4) enthalten sind, elektrisch steuerbar ist.

## Claims

1. Total artificial heart comprising, on the one hand, the indissociable functional unit constituted by:

– a pericardial module (3) adapted to be housed in the cavity (2) of the natural heart (1) to be replaced and enclosed in a tight envelope (8) presenting only three orifices (25, 26 and 29) for connection respectively to the right atrium OD, the pulmonary artery AP and the left atrium OG, said orifices (25 and 26) for connection to the right atrium OD and to the pulmonary artery AP being provided with valves to serve respectively as inlet orifice and outlet orifice for a first pump (13, 14) housed in said envelope (8) and intended to perform the function of the right-hand part of the natural heart to be replaced;

– an extra-pericardial module (4) adapted to be housed in a space in the abdominal cavity of the receiver patient and to perform the function of the left-hand part of the natural heart (1) to be replaced, this extra-pericardial module comprising a second pump (50, 52) enclosed in a tight envelope (40) provided with an inlet orifice (53) and an outlet orifice (56), each provided with a valve, said outlet orifice (56) being adapted to be connected to the abdominal part of the aorta;

– a functional link (5) between said modules comprising only:

. a first tube (33) passing through said envelope (8) of said pericardial module (3) and joining the orifice (29) of the latter corresponding to the left atrium OG and the inlet orifice (53) of the second pump (50, 52) incorporated in said extra-pericardial module (4);

. a second tube (32) establishing a gaseous communication between the sides of said first and second pumps opposed to the blood passing therethrough ; and, on the other hand, a control device (89, 90, 92, 93) which actuates said pumps in opposition.

2. Total artificial heart according to claim 1, characterized in that said pericardial module (3) encloses an electro-mechanical system for actuating said first pump (13, 14).

3. Total artificial heart according to one of claims 1 or 2, characterized in that said envelope (8) of said pericardial module (3) presents at least approximately the form, volume and mass of the natural heart to be replaced, and in that, on said envelope (8), the arrangement of the orifices (25, 26 and 29) for connection to the right atrium OD, the pulmonary artery AP and the left atrium OG corresponds at least substantially to the natural arrangement of these atria and artery.

4. Total artificial heart according to one of claims 1 to 3, characterized in that, in said pericardial module (3), the valves provided in the orifices (25, 26) for connection to the right atrium and pulmonary artery are of the electro-controlled type.

5. Total artificial heart according to one of claims 1 to 4, characterized in that said first pump (13, 14) is of the diaphragm and pusher-plate type.

6. Total artificial heart according to any one of claims 1 to 5, characterized in that said link (5) connects the lower part of said envelope (8) of said pericardial module (3) to the upper part of said envelope (40) of said extra-pericardial module (4).

7. Total artificial heart according to any one of claims 1 to 6, characterized in that said second tube (32) of said link (5) between the modules is longitudinally supple but radially rigid and encloses said first tube (33).

8. Total artificial heart according to any one of claims 1 to 7, characterized in that said second pump (50, 52) is of the diaphragm and pusher-plate type.

9. Total artificial heart according to any one of claims 1 to 8, characterized in that each of the two valves (54, 57) incorporated in said extra-pericardial module (4) is of the electro-controlled type.

*Fig.1*

*Fig.3*

*Fig.2*

EP 0 227 537 B1

*Fig.4*

*Fig.5*

*Fig.6*